# EUROPEAN PATENT APPLICATION

(11) **EP 3 266 374 A1**
(43) Date of publication of application: **10.01.2018**
(21) Application number: 15884010.8
(22) Date of filing: 06.11.2015
(51) Int. Cl.: A61B 5/11, A61B 5/22, A61B 5/00, G08B 31/00

(54) **CONDITION DETECTION METHOD, CONDITION DETECTION DEVICE, AND CONDITION DETECTION PROGRAM**

(30) Priority: 03.03.2015 JP 2015041812
(71) Applicant: Fujitsu Limited, Kawasaki-shi, Kanagawa 211-8588 (JP)
(72) Inventor: WASHIZAWA, Shiho, Kawasaki-shi Kanagawa 211-8588 (JP); MAEDA, Kazuho, Kawasaki-shi Kanagawa 211-8588 (JP); INOMATA, Akihiro, Hayes Middlesex UB4 8FE (JP)
(74) Representative: Haseltine Lake LLP
(86) International application number: PCT/JP2015/081414
(87) International publication number: WO 2016/139844

(57) **Abstract**

A state detection device (1) stores first information obtained from an environment sensor and second information obtained from a motion sensor in a predetermined segment in a data recording DB (21). The state detection device (1) specifies a behavior segment indicating a segment in which a subject performs a specific behavior and a non-behavior segment indicating a segment in which the subject does not perform the specific behavior using the second information stored in the data recording DB (21). The state detection device (1) determines a feature amount to be used for detecting a specific state of the subject using respective values of a plurality of feature amounts included in the first information stored in the data recording DB (21) in each of the behavior segment and the non-behavior segment and determines a threshold value for the determined feature amount, for distinguishing a behavior and a non-behavior. The state detection device (1) is configured to detect the specific state of the subject from the information obtained from the environment sensor using the determined feature amount and the determined threshold value. In this way, it is possible to detect the behavior of the subject depending on the situation of the subject when detecting the subject's behavior.

## Description

### [Technical Field]

The present invention relates to a state detection method and the like.

### [Background Art]

In recent years, motion sensors are used for detecting behaviors in daily life. A technique for comparing detection data with a threshold of learning data obtained in advance for each behavior and detecting a behavior of a subject is known in relation to the motion sensor (for example, Non Patent Document 1).

### [Citation List]

### [Non Patent Citation]

### Non Patent Document

Non Patent Document 1: O.D. Lara et al., "A Survey on Human Activity Recognition using Wearable Sensors", IEEE Communications Survey & Tutorials, vol. 15, no. 3, pp. 1192-1209, 2013

### [Summary of Invention]

### [Technical Problem]

However, in a conventional behavior detection technique, there is a problem that it is difficult to detect a behavior depending on the situation of a subject when detecting the behavior of the subject. That is, when the situation of a subject is the same as that during learning, by comparing the detection data with the threshold obtained by learning, it is possible to detect the behavior. However, when the situation of the subject is different from that during learning, there is a possibility that an error occurs in the behavior detection or the behavior is not detected.

Here, a human behavior changes depending on a growth phase such as children, adults, and elderly people. The human behavior changes depending on a physical condition, a time period, and a recovery phase of an illness even if the behavior is of the same person. As an example, when a person has a back injury, the behavior such as walking or sitting is slower than when the person does not have a back injury. As another example, immediately after a person is discharged from a hospital, since the body does not move as intended, the behavior is slow. As described above, when the situation of the subject is different from that during learning, there is a possibility that an error occurs in the behavior detection or the behavior is not detected.

An object of one aspect is to detect a behavior of a subject depending on the situation of the subject when detecting the behavior of the subject.

### [Solution to Problem]

According to one aspect of the present application, a computer executes processes including: storing first information obtained from a first sensor and second information obtained from a second sensor in a predetermined segment in a storage unit; specifying a behavior segment indicating a segment in which a subject performs a specific behavior and a non-behavior segment indicating a segment in which the subject does not perform the specific behavior using the second information stored in the storage unit; determining a feature amount to be used for detecting a specific state of the subject using respective values of a plurality of feature amounts included in the first information stored in the storage unit in each of the behavior segment and the non-behavior segment and determining a threshold value for the determined feature amount, for distinguishing a behavior and a non-behavior; and detecting the specific state of the subject from the information obtained from the first sensor using the determined feature amount and the determined threshold value.

### [Advantageous Effects of Invention]

According to one aspect, it is possible to detect a behavior of a subject depending on the situation of the subject when detecting the behavior of the subject.

### [Brief Description of Drawings]

FIG. 1 is a functional block diagram illustrating a configuration of a state detection device according to a first embodiment.
FIG. 2 is a diagram illustrating an example of the flow of a state detection process according to the first embodiment.
FIG. 3 is a diagram illustrating an example of a data structure of a data recording DB according to the first embodiment.
FIG. 4 is a diagram illustrating an example of installation of environment sensors.
FIG. 5 is a flowchart illustrating a processing procedure of a state detection process according to the first embodiment.
FIG. 6 is a functional block diagram illustrating a configuration of a state detection device according to a second embodiment.
FIG. 7 is a diagram illustrating an example of a first consistency determination process according to the second embodiment.
FIG. 8 is a diagram illustrating an example of a second consistency determination process according to the second embodiment.
FIG. 9 is a diagram illustrating an example of a third consistency determination process according to the second embodiment.
FIG. 10 is a diagram illustrating an example of a change extraction process according to the second embodiment.
FIG. 11 is a flowchart illustrating a processing procedure of a segment specifying process according to the second embodiment.
FIG. 12A is a diagram illustrating a first specific example of a consistency determination process according to the second embodiment.
FIG. 12B is a diagram illustrating a second specific example of the consistency determination process according to the second embodiment.
FIG. 12C is a diagram illustrating a third specific example of the consistency determination process according to the second embodiment.
FIG. 13A is a diagram illustrating a first specific example of a change extraction process according to the second embodiment.
FIG. 13B is a diagram illustrating a second specific example of the change extraction process according to the second embodiment.
FIG. 13C is a diagram illustrating a third specific example of the change extraction process according to the second embodiment.
FIG. 14 is a functional block diagram illustrating a configuration of a state detection device according to a third embodiment.
FIG. 15 is a diagram illustrating an example of a threshold value estimation process according to the third embodiment.
FIG. 16 is a flowchart illustrating a processing procedure of the threshold value estimation process according to the third embodiment.
FIG. 17 is a diagram illustrating an example of a computer that executes a state detection program.

### [Embodiments for Carrying Out the Invention]

Hereinafter, embodiments of a state detection method, a state detection device, and a state detection program disclosed in the present application will be described in detail with reference to the drawings. The present invention is not limited to these embodiments. Moreover, a segment in embodiments indicates a segment on a time axis.

### First Embodiment

### [Configuration of State Detection Device]

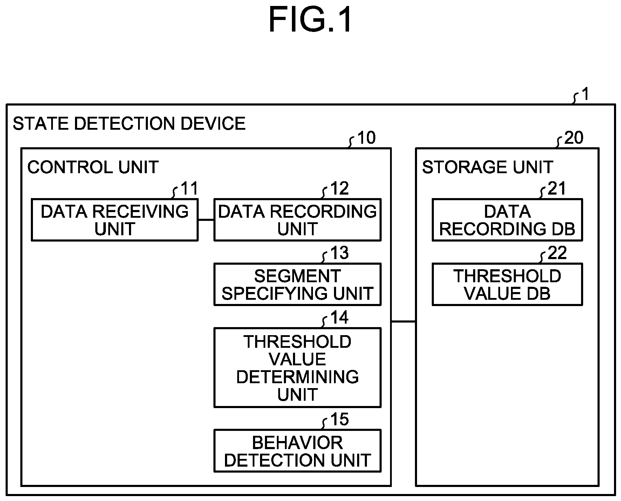

FIG. 1 is a functional block diagram illustrating a configuration of a state detection device according to a first embodiment. A state detection device 1 according to the first embodiment specifies a behavior segment indicating a segment in which a behavior of a subject corresponding to a behavior type that is to be detected is performed and a non-behavior segment indicating a segment in which the behavior is not performed using data indicating a change in a behavior of a person, obtained from environment sensors installed in a closed environment space. The state detection device 1 determines a feature amount and a threshold value used for detecting the behavior of a person using data indicating the information on the behavior of the person obtained from a motion sensor attached to the person in the specified behavior segment and the specified non-behavior segment. Hereinafter, a case in which the behavior type is "walking" will be described.

The closed environment space mentioned herein indicates a residential environment space, for example, and the closed environment space may be a space in which it is possible to understand the number of persons present in the space using data obtained from environment sensors. Hereinafter, a case in which the closed environment space is a residential environment space will be described.

The environment sensor mentioned herein is a sensor that measures a surrounding environment, a sensor that measures the state of an installed object itself, and a sensor that measures the state of an entire residential environment. The sensor that measures the surrounding environment may be, for example, a luminance sensor, a lighting sensor, a thermo sensor, a temperature sensor, a thermal bubble sensor, a lighting sensor, an electricity sensor, and a human sensor, but is not limited to this. The sensor that measures an installed object itself may be, for example, a blind sensor, a door sensor, a bed sensor, and a window sensor, but is not limited to this. The sensor that measures the state of the entire residential environment may be, for example, a water consumption sensor, an electricity consumption sensor, and a gas consumption sensor, but is not limited to this.

The motion sensor mentioned herein is a sensor that measures a motion of a person and may be an acceleration sensor or a gyro sensor, for example, but is not limited to this.

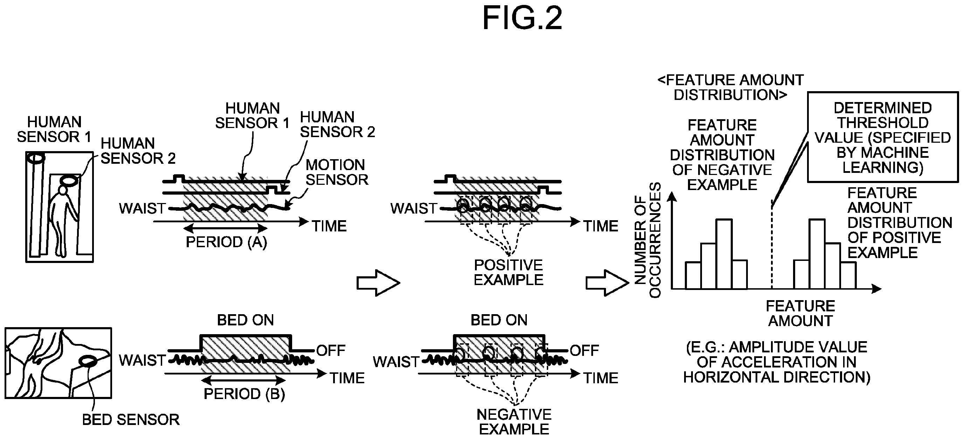

Here, the flow of a state detection process of the state detection device 1 will be described with reference to FIG. 2. FIG. 2 is a diagram illustrating an example of the flow of a state detection process. As illustrated on the left side of FIG. 2, the state detection device 1 acquires data indicating a change in a behavior of a person (in this example, data obtained from human sensors 1 and 2) and specifies a period (a behavior segment) in which the person walks and a period (a non-behavior segment) in which the person does not walk based on information on a time point at which the data changes. In this example, it is assumed that the human sensors 1 and 2 and a bed sensor are installed in a residential environment space. The state detection device 1 specifies a period between a time point at which the value of the human sensor 1 changes from OFF to ON and a time point at which the value of the human sensor 2 changes from OFF to ON as a behavior segment. The state detection device 1 specifies a period between a time point at which the bed sensor changes from OFF to ON and a time point at which the bed sensor changes from ON to OFF as a non-behavior segment.

As illustrated at the center of FIG. 2, the state detection device 1 extracts data obtained from the motion sensor so that the data obtained in the behavior segment is positive example data and the data obtained in the non-behavior segment is negative example data. In this example, the positive example data and the negative example data are extracted from the motion sensor attached to the waist of a subject.

As illustrated on the right side of FIG. 2, the state detection device 1 extracts feature amounts such as a peak interval and a peak amplitude from the extracted positive example data and the extracted negative example data. The state detection device 1 determines a feature amount and a threshold value to be used for detecting behaviors from a plurality of extracted feature amounts according to a machine learning algorithm. That is, the state detection device 1 determines a feature amount for distinguishing a feature amount distribution of the positive example from a feature amount distribution of the negative example using machine learning. That is, the state detection device 1 determines such a feature amount that the feature amount distribution of the positive example does not overlap with the feature amount distribution of the negative example. Moreover, the state detection device 1 determines a threshold value for distinguishing behaviors from non-behaviors with respect to the determined feature amount. In this example, an amplitude value of an acceleration in a horizontal direction is an example of the feature amount. After that, the state detection device 1 detected walking as a behavior type of a subject from the information obtained from the motion sensor using the determined feature amount and the determined threshold value.

Returning to FIG. 1, the state detection device 1 includes a control unit 10 and a storage unit 20.

The storage unit 20 corresponds to a storage device of a nonvolatile semiconductor memory such as a flash memory or ferroelectric random access memory (FRAM) (registered trademark), for example. The storage unit 20 includes a data recording database (DB) 21 and a threshold value DB 22. The data obtained from the environment sensors and the data obtained from the motion sensor are stored in the data recording DB 21 in a time-series order. The respective items of data are recorded in the data recording DB 21 by a data recording unit 12 to be described later. A data structure of the data recording DB 21 will be described later.

A threshold value and a feature amount are stored in the threshold value DB 22. The threshold value and the feature amount are recorded in the threshold value DB 22 by a threshold value determining unit 14 to be described later. When the state detection device 1 detects behaviors of a plurality of behavior types, the threshold value and the feature amount of each behavior type may be stored in the threshold value DB 22.

The control unit 10 includes an internal memory for storing a program and control data that define various processing procedures and executes various processes using the program and the control data. The control unit 10 corresponds to an electronic circuit of an integrated circuit such as an application specific integrated circuit (ASIC) or a field programmable gate array (FPGA). Alternatively, the control unit 10 corresponds to an electronic circuit such as a central processing unit (CPU) or a micro processing unit (MPU). Furthermore, the control unit 10 includes a data receiving unit 11, a data recording unit 12, a segment specifying unit 13, a threshold value determining unit 14, and a behavior detection unit 15.

The data receiving unit 11 receives the data obtained from the environment sensors and the data obtained from the motion sensor.

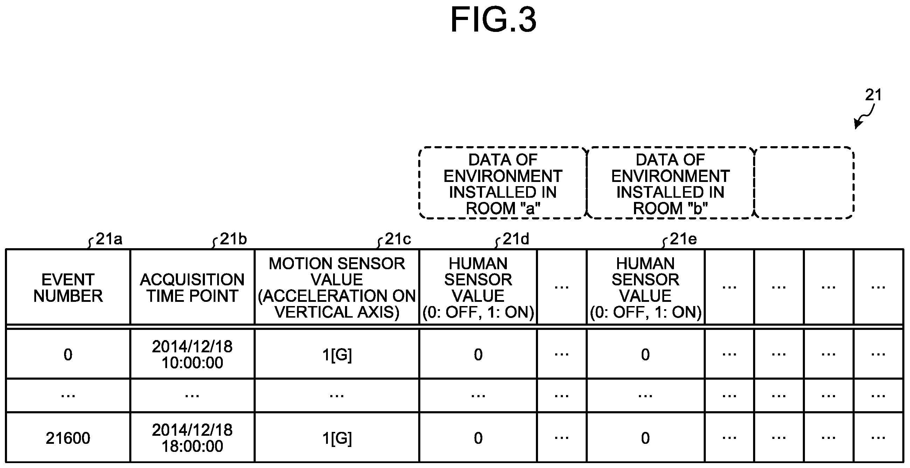

The data recording unit 12 records various items of data received by the data receiving unit 11 in the data recording DB 21. The data recording unit 12 records various items of data for a predetermined segment in the data recording DB 21. The predetermined segment is one day, for example, but is not limited to this and may be a half day or a 3/4 day. That is, the predetermined segment may be a segment in which a threshold value can be determined. Hereinafter, a case in which the predetermined segment is one day will be described. Here, the data structure of the data recording DB 21 will be described with reference to FIG. 3. FIG. 3 is a diagram illustrating an example of the data structure of the data recording DB according to the first embodiment.

As illustrated in FIG. 3, the data recording DB 21 stores an acquisition time 21b and a motion sensor value (acceleration on a vertical axis) 21c in correlation with an event number 21a. Furthermore, the data recording DB 21 stores a human sensor value 21d as the data of an environment sensor installed in a room "a" and a human sensor value 21e as the data of an environment sensor installed in a room "b" in correlation with the event number 21a.

The event number 21a is a number assigned to each event. The event number 21a is assigned with a serial number such that the earlier an event, the smaller the event number. The acquisition time 21b is a time point at which an event corresponding to the event number 21a was acquired. The motion sensor value (the acceleration on the vertical axis) 21c is a value of an acceleration sensor attached to the waist of a user, for example, and is a value of acceleration on the vertical axis. The acceleration on the vertical axis expressed by the motion sensor value mentioned herein is an example of a feature amount. Although the acceleration value on the vertical axis is mentioned as an example of the motion sensor value, the motion sensor value is not limited to this and may be an amplitude value of an acceleration in a horizontal direction or may be an amplitude value on each axis of a gyro sensor.

The human sensor values 21d and 21e are OFF (0), for example, when no person is detected and are ON (1), for example, when a person is detected. The human sensor value 21d is the data of an environment sensor installed in the room "a", for example, included in the residential environment space used when determining the threshold value. The human sensor value 21e is the data of an environment sensor installed in the room "b", for example, included in the residential environment space used when determining the threshold value. The human sensor values 21d and 21e are examples of the environment sensor data installed in the rooms "a" and "b", and the environment sensor data is not limited to this. The environment sensor value may be set according to the environment sensor installed in the room "a". The environment sensor value may be set according to the environment sensor installed in the room "b". Moreover, the environment sensor data is not limited to the data of the environment sensor installed in the room "a" and the data of the environment sensor installed in the room "b" but may be the data of an environment sensor installed in a room included in the residential environment space.

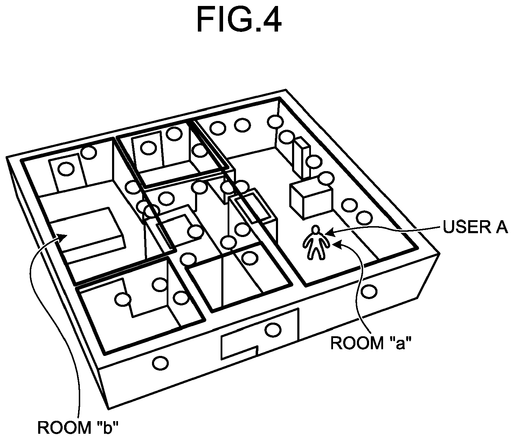

FIG. 4 is a diagram illustrating an example of installation of environment sensors. As illustrated in FIG. 4, a residential environment space used when determining the threshold value is depicted. In this example, the rooms "a" and "b" and other rooms are included in the residential environment space. Environment sensors are installed at positions indicated by circles in the respective rooms. A user A has a motion sensor attached to the waist, for example.

Returning to FIG. 1, the segment specifying unit 13 specifies a behavior segment and a non-behavior segment of a subject using the environment sensor values stored in the data recording DB 21. For example, the segment specifying unit 13 specifies a period between a time point at which the human sensor value changes from OFF (0) to ON (1) and a time point at which the human sensor value changes from ON (1) to OFF (0) as a behavior segment using the human sensor values stored in the data recording DB 21. The segment specifying unit 13 specifies a period between a time point at which the bed sensor value changes from OFF (0) to ON (1) and a time point at which the human sensor value changes from (1) to OFF (0) as a non-behavior segment using the bed sensor values stored in the data recording DB 21.

The threshold value determining unit 14 determines a feature amount and a threshold value used for detecting walking of a subject using the respective values of the respective feature amounts of the motion sensor stored in the data recording DB 21 in the behavior segment and the non-behavior segment. For example, the threshold value determining unit 14 extracts the data in the behavior segment from the respective values of the respective feature amounts of the motion sensor stored in the data recording DB 21 as positive example data. The threshold value determining unit 14 extracts the data in the non-behavior segment from the respective values of the respective feature amounts of the motion sensor stored in the data recording DB 21 as negative example data. Moreover, the threshold value determining unit 14 generates a feature amount distribution of the positive example and a feature amount distribution of the negative example for the respective feature amounts from the positive example data and the negative example data of the respective feature amounts according to a machine learning algorithm. The threshold value determining unit 14 determines a feature amount for distinguishing the feature amount distribution of the positive example from the feature amount distribution of the negative example and determines a threshold value for distinguishing behaviors from non-behaviors with respect to the determined feature amount. The threshold value determining unit 14 records the determined threshold value and the determined feature amount in the threshold value DB 22. In this way, the threshold value determining unit 14 can determine a threshold value depending on the current situation of the subject using the latest one-day data stored in the data recording DB 21.

The behavior detection unit 15 detects walking of the subject using the threshold value and the feature amount stored in the threshold value DB 22 from the information obtained from the motion sensor. In this way, the behavior detection unit 15 can detect walking as a behavior type depending on the current situation of the subject.

### [Flowchart of State Detection Process]

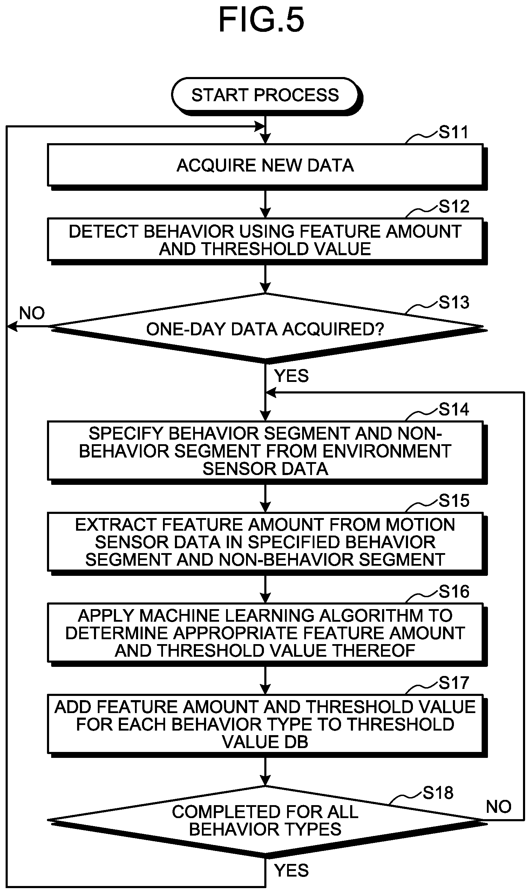

Next, an example of a processing procedure of the state detection process according to the first embodiment will be described with reference to FIG. 5. FIG. 5 is a flowchart illustrating a processing procedure of the state detection process according to the first embodiment. A feature amount and a threshold value to be used when detecting a behavior for each behavior type of the subject are stored in the threshold value DB 22.

First, upon acquiring new one-day data (that is, the data obtained from environment sensors and the data obtained from the motion sensor) (step S11), the data receiving unit 11 records the acquired data in the data recording DB 21.

The behavior detection unit 15 detects the behavior of the subject using the feature amount and the threshold value stored in the threshold value DB 22 from the data obtained from the motion sensor (step S12).

The data recording unit 12 determines whether one-day data has been acquired (step S13). When it is determined that one-day data has not been acquired (step S13: No), the data recording unit 12 proceeds to step S11 in order to acquire new data.

On the other hand, when it is determined that one-day data has been acquired (step S13: Yes), the segment specifying unit 13 specifies a behavior segment and a non-behavior segment of the subject from the environment sensor data stored in the data recording DB 21 (step S14). For example, the segment specifying unit 13 specifies a period between a time point at which the human sensor values changes from OFF (0) to ON (1) and a time point at which the human sensor value changes from ON (1) to OFF (0) as a behavior segment using the human sensor values stored in the data recording DB 21. The segment specifying unit 13 specifies a period between a time point at which the bed sensor value changes from OFF (0) to ON (1) and a time point at which the human sensor value changes from ON (1) to OFF (0) as a non-behavior segment using the bed sensor values stored in the data recording DB 21.

Subsequently, the threshold value determining unit 14 extracts a feature amount from the motion sensor data stored in the data recording DB 21 in the behavior segment and the non-behavior segment specified by the segment specifying unit 13 (step S15). The motion sensor data mentioned herein means the motion sensor values stored in the data recording DB 21.

The threshold value determining unit 14 determines an appropriate feature amount and a threshold value thereof from the extracted feature amounts according to a machine learning algorithm (step S16). For example, the threshold value determining unit 14 extracts the behavior segment data as positive example data from the respective values of the respective feature amounts of the motion sensor stored in the data recording DB 21. The threshold value determining unit 14 extracts the non-behavior segment data as negative example data from the respective values of the respective feature amounts of the motion sensor stored in the data recording DB 21. The threshold value determining unit 14 generates a positive example feature amount distribution and a negative example feature amount distribution for the respective feature amounts from the positive example data and the negative example data of the respective feature amounts according to the machine learning algorithm. The threshold value determining unit 14 determines a feature amount for distinguishing the positive example feature amount distribution from the negative example feature amount distribution and determines a threshold value for the determined feature amount, for distinguishing behaviors from non-behaviors.

Subsequently, the threshold value determining unit 14 adds the feature amount and the threshold value for each behavior type to the threshold value DB 22 (step S17). The threshold value determining unit 14 determines whether all behavior types have been processed (step S18). When it is determined that all behavior types have not be processed (step S18: No), the threshold value determining unit 14 proceeds to step S14 so that a non-processed behavior type is processed.

On the other hand, when it is determined that all behavior types have been processed (step S18: Yes), the threshold value determining unit 14 proceeds to step S11 so that the next one-day data is processed.

### [Advantages of First Embodiment]

In this way, in the first embodiment, the state extraction device 1 records the data obtained from the motion sensor and the data obtained from the environment sensors in a predetermined segment in the data recording DB 21. The state extraction device 1 specifies a behavior segment indicating a segment in which a subject has performed a specific behavior and a non-behavior segment indicating a segment in which the subject has not performed the specific behavior using the data obtained from environment sensors, stored in the data recording DB 21. The state extraction device 1 determines a feature amount to be used for detecting a specific state of the subject using the respective values of a plurality of feature amounts included in the data stored in the data recording DB 21, obtained from the motion sensor in each of the behavior segment and the non-behavior segment. Furthermore, the state detection device 1 determines a threshold value for the determined feature amount, for distinguishing behaviors from non-behaviors. The state extraction device 1 detects the specific state of the subject using the determined feature amount and the determined threshold value from the data obtained from the motion sensor. According to this configuration, the state detection device 1 can determine a threshold value of the specific state depending on the current situation of the subject using the latest data for a predetermined segment stored in the data recording DB 21. As a result, the state detection device 1 can detect a specific state of the subject (for example, the behavior of the subject) depending on the current situation of the subject using the motion sensor only. As an example, the state detection device 1 can detect the behavior of the subject depending on the current situation of the subject even when the symptom of the recovery stage or the like of an illness of the subject changes day by day.

### Second Embodiment

It has been described that the state detection device 1 according to the first embodiment combines the data of a plurality of environment sensors installed in a residential environment space to specify a behavior segment and a non-behavior segment of the subject's behavior. However, the state detection device 1 may erroneously detect the behavior segment and the non-behavior segment of the subject when another source (for example, a cohabitant or a visitor) other than the subject is present. Therefore, the state detection device 1 according to a second embodiment combines a change in the data of a plurality of environment sensors installed in the residential environment space and the data of the motion sensor of the subject to select the environment sensor data in which the change is associated with a motion of the subject. Moreover, the state detection device 1 may combine the changes in the selected data to reliably specify the behavior segment and the non-behavior segment of the subject in relation to the subject's behavior.

Therefore, the state detection device 1 according to the second embodiment combines a change in the data of a plurality of environment sensors installed the residential environment space and the motion sensor data of the subject to select the environment sensor data in which the change is associated with a motion of the subject. Moreover, a case in which the state detection device 1 combines changes in the selected data to reliably specify the behavior segment and the non-behavior segment of the subject in relation to the subject's behavior will be described.

### [Configuration of State Detection Device according to the second embodiment]

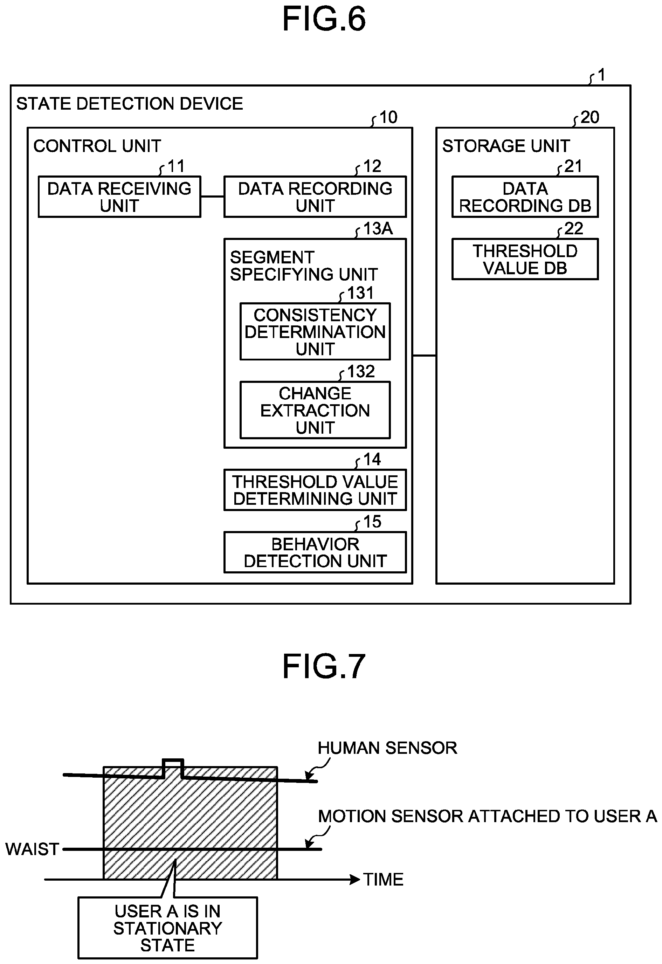

FIG. 6 is a functional block diagram illustrating a configuration of a state detection device according to the second embodiment. The same constituent elements as those of the state detection device 1 illustrated in FIG. 1 are denoted by the same reference numerals, and the redundant description of the constituent elements and operations will not be provided. A difference between the first embodiment and second embodiment is that the segment specifying unit 13 is changed to a segment specifying unit 13A and a consistency determination unit 131 and a change extraction unit 132 are added to the segment specifying unit 13A.

The segment specifying unit 13A estimates the number of persons present in the residential environment space and specifies a behavior segment and a non-behavior segment of a subject using only a change in the data of an environment sensor that does not react to a person other than the subject.

The consistency determination unit 131 determines whether there is a consistency between the motion sensor data of the subject and a change in the data of all environment sensors present in the residential environment space. In other words, the consistency determination unit 131 estimates the number of persons present in the residential environment space by determining consistency among the data obtained from sensors in each segment between closing of a door sensor installed at a position corresponding to an entrance hall of the residential environment space and the subsequent opening of the door sensor. For example, the consistency determination unit 131 sets the number of persons present in the residential environment space to "1" by assuming that only the subject is present therein. The consistency determination unit 131 determines whether there is a consistency between the motion sensor data of the subject and changes in the data of all environment sensors using the data stored in the data recording DB 21. When it is determined that there is no consistency, the consistency determination unit 131 determines that there is a person other than the subject to which the environment sensor reacts and adds "1" to the number of persons in the residential environment space.

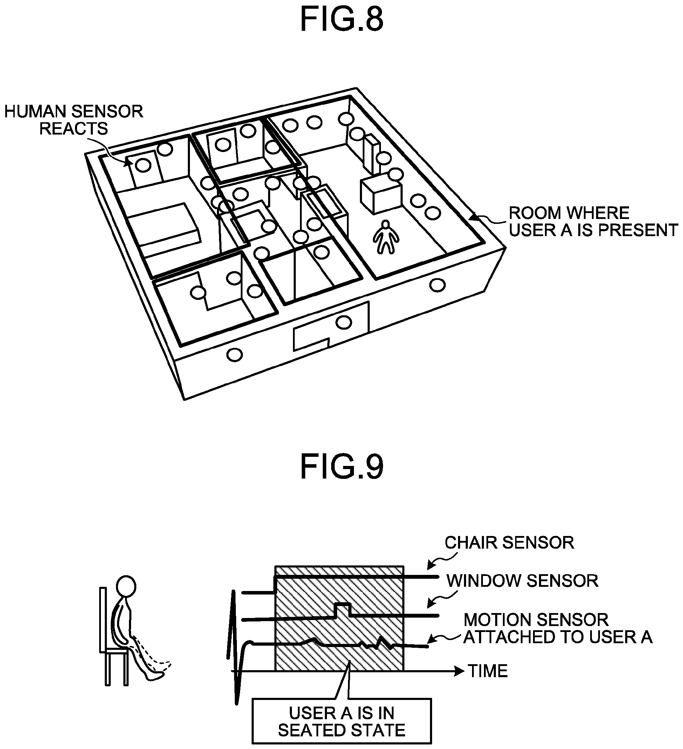

Here, a case in which it is determined that there is no consistency by the consistency determination process of the consistency determination unit 131 will be described with reference to FIGS. 7 to 9. FIG. 7 illustrates a case in which there is no consistency and there is a change in the environment sensor data when there is no reaction of the motion sensor. FIG. 8 illustrates a case in which there is no consistency and there is a change in the data of an environment sensor in a room different from the room where the subject is present. FIG. 9 illustrates a case in which there is no consistency and there is a change in the data of the environment sensor in a room where the subject is present even when the subject does not stand up after being seated. In FIGS. 7 to 9, the subject will be denoted by "A". The human sensor data and the data of a motion sensor attached to the waist of the subject A are stored in the data recording DB 21.

FIG. 7 illustrates a change in the data of a human sensor installed in a certain room in a certain segment and the data of the motion sensor attached to the waist of the subject A in the same segment. The consistency determination unit 131 determines whether there is a consistency between the motion sensor data and the changes in the data of all environment sensors using the data stored in the data recording DB 21. In this example, although the human sensor data changes from OFF to ON, the motion sensor data of the subject A indicates a stationary state. Therefore, since there is a change in the human sensor data when there is no reaction of the motion sensor of the subject A, the consistency determination unit 131 determines that there is no consistency between the human sensor and a sensor which reacts to the subject A. That is, the consistency determination unit 131 determines that there is another person other than the subject A and adds "1" to the number of persons.

As illustrated in FIG. 8, a case in which a room where the subject A is present is different from a room where an environment sensor reacts is depicted. The consistency determination unit 131 determines whether there is a consistency between the motion sensor data and the changes in the data of all environment sensors using the data stored in the data recording DB 21. In this example, the consistency determination unit 131 specifies a room where the subject A is present from the information on a room associated with a change in the data of an environment sensor that specifies a latest behavior of the subject A using the data stored in the data recording DB 21. Moreover, since the environment sensor in a room different from the room where the subject A is present reacts, the consistency determination unit 131 determines that there is no consistency between the human sensor and a sensor which reacts to the subject A. That is, the consistency determination unit 131 determines that there is another person other than the subject A and adds "1" to the number of persons.

As illustrated in FIG. 9, a change in the data of a chair sensor installed in a certain room and a change in the data of a window sensor in a certain segment, and the data of a motion sensor attached to the waist of the subject A in the same segment are depicted. The consistency determination unit 131 determines whether there is a consistency between the motion sensor data and the changes in the data of all environment sensors using the data stored in the data recording DB 21. In this example, the motion sensor data of the subject A in a certain segment indicates a sitting state and the chair sensor data indicates the ON state. In the same segment, the window sensor data changes from OFF to ON. Therefore, the consistency determination unit 131 determines that there is no consistency. That is, a person may sometimes perform a specific behavior after performing a certain behavior like the way that a person "stands up" after being "seated" and then walks. Since there is a change in the window sensor even if the subject A does not "stand up" after being "seated", the consistency determination unit 131 determines that there is no consistency between the window sensor and a sensor which reacts to the subject A. That is, the consistency determination unit 131 determines that there is another person other than the subject A and adds "1" to the number of persons.

Returning to FIG. 6, the change extraction unit 132 extracts a change in the data of an environment sensor that does not react to any person other than the subject. "A sensor that does not react to any person other than the subject" means that it is determines that there is no consistency if the sensor reacts to any person other than the subject. In other words, the change extraction unit 132 extracts a change in the data of the environment sensor that reacts to the subject only. For example, the change extraction unit 132 determines whether there is a consistency between the data of a motion sensor of a person other than the subject and changes in the data of all environment sensors for persons other than the subject using the data stored in the data recording DB 21. The process of determining whether there is a consistency is the same as the consistency determination process of the consistency determination unit 131. The change extraction unit 132 extracts a change in the data of the environment sensor for which it is determined that there is no consistency for all persons other than the subject.

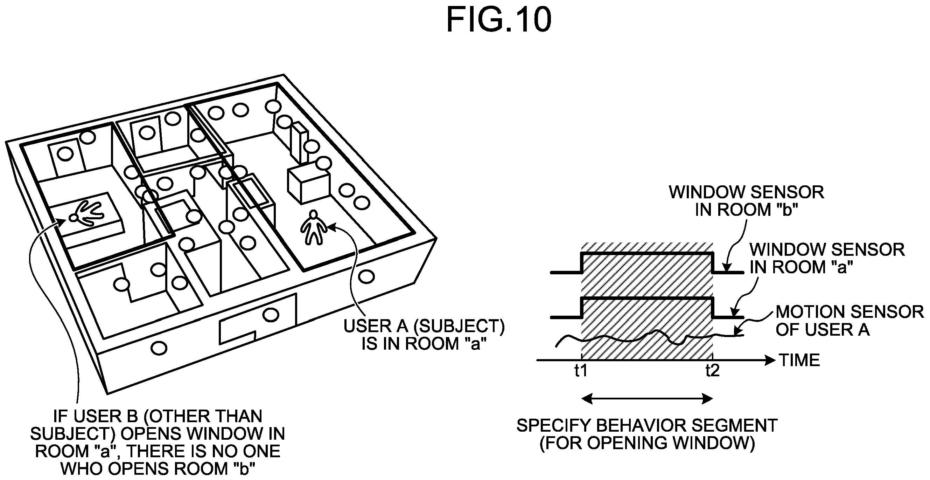

Here, an example of the change extraction process of the change extraction unit 132 will be described with reference to FIG. 10. FIG. 10 is a diagram illustrating an example of the change extraction process according to the second embodiment. In FIG. 10, the subject will be denoted by "A" and a person other than the subject will be denoted by "B". The window sensor data and the data of the motion sensor attached to the waist of the subject A are recorded in the data recording DB 21.

The change extraction unit 132 specifies a room where the subject A is present as "A" as illustrated on the left side of FIG. 10 from the information on a room associated with a change in the data of an environment sensor for specifying the latest behavior of the subject A using the data stored in the data recording DB 21.

As illustrated on the right side of FIG. 10, a change in the data of a window sensor installed in the room "b" and a change in the data of a window sensor installed in the room "a" in a certain segment and the motion sensor data of the subject A are depicted. The change extraction unit 132 determines whether there is a consistency in the changes in the data of all environment sensors for persons other than the subject using the data stored in the data recording DB 21. In this example, if the window sensor installed in the room "a" where the subject is present reacts to the person B, since the person to which the window sensor installed in the room "b" reacts is not present in the room "b", the change extraction unit 132 determines that there is no consistency between the window sensor in the room "b" and a sensor that reacts to the person B. Therefore, the change extraction unit 132 extracts a change in the data of the window sensor in the room "a", for which it is determined that the window sensor data is not consistent with the person B. In other words, the change extraction unit 132 extracts a change in the data of the window sensor in the room "a", which reacts to the subject A only. In this example, a change in the data of the window sensor in the room at time points t1 and t2 is extracted.

After that, the segment specifying unit 13A can specify a behavior segment in which a person opens a window using the extracted change in the data of the window sensor in the room "a".

### [Flowchart of Segment Specifying Process]

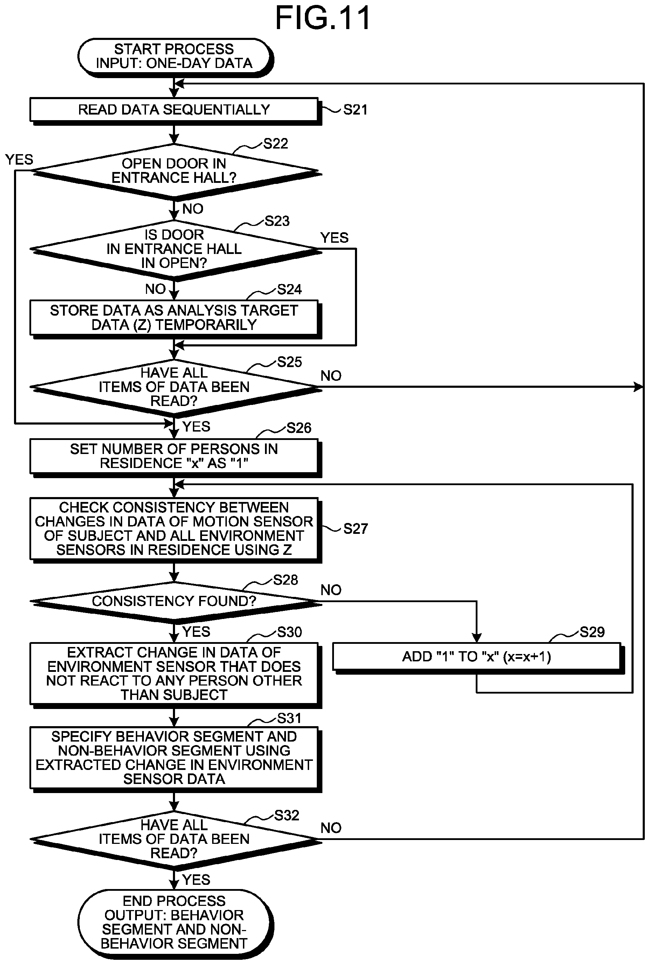

Next, an example of a processing procedure of a segment specifying process according to the second embodiment will be described with reference to FIG. 11. FIG. 11 is a flowchart illustrating a processing procedure of a segment specifying process according to the second embodiment. A processing procedure of an entire state detection process including the segment specifying process is the same as that described in FIG. 5, and the description thereof will not be provided. An example of S14 in the processing procedure of the state detection process described in FIG. 5 is a processing procedure of the segment specifying process illustrated in this diagram.

In this example, it is assumed that the segment specifying unit 13A receives one-day data. The data mentioned herein means the environment sensor data and the motion sensor data stored in the data recording DB 21. Moreover, it is assumed in FIG. 11 that the residential environment space is a residence and a door sensor is installed in the entrance hall of the residence.

The segment specifying unit 13A sequentially read the input one-day data (step S21). The segment specifying unit 13A determines whether the read data indicates an open state of the door in the entrance hall (step S22). That is, the segment specifying unit 13A determines whether the data of the door sensor installed in the entrance hall changes from ON indicating a closed state to OFF indicating an open state.

When it is determined that the read data does not indicate an open state of the door in the entrance hall (step S22: No), the segment specifying unit 13A determines whether the read data indicates an open state of the door in the entrance hall (step S23). That is, the segment specifying unit 13A determines whether the data of the door sensor installed in the entrance hall is OFF. When it is determined that the read data indicates an open state of the door in the entrance hall (step S23: Yes), the segment specifying unit 13A does not store the data and proceeds to step S25. This data is not stored since it is not possible to estimate the number of persons present in the residence when the door in the entrance hall is open.

On the other hand, when it is determined that the read data does not indicate an open state of the door in the entrance hall (step S23: No), the segment specifying unit 13A stores the data temporarily in the storage unit 20 as data to be analyzed (step S24). This data is stored since it is possible to estimate the number of persons present in the residence when the door in the entrance hall is closed. After that, the segment specifying unit 13A proceeds to step S25.

In step S25, the segment specifying unit 13A determines whether all items of the input one-day data have been read (step S25). When it is determined that all items of the input one-day data have not been read (step S25: No), the segment specifying unit 13A proceeds to step S21 so that the next data is read. On the other hand, when it is determined that all items of the input one-day data have been read (step S25: Yes), the segment specifying unit 13A proceeds to step S26.

In step S22, when it is determined that the read data indicates an open state of the door in the entrance hall (step S22: Yes), the consistency determination unit 131 sets the number of persons x present in the residence to "1" (step S26). Here, "x" is a variable indicating the number of persons present in the residence.

The consistency determination unit 131 checks a consistency between the data of the motion sensor of the subject and the changes in the data of all environment sensors in the residence using the data stored temporarily (step S27). In other words, the consistency determination unit 131 checks a consistency between items of data obtained from sensors in a segment after the door sensor installed in the entrance hall of the residence is closed before the door is open again.

The consistency determination unit 131 determines whether there is a consistency between the motion sensor data of the subject and the changes in the data of all environment sensors in the residence (step S28). When it is determined that there is no consistency between the motion sensor data of the subject and the changes in the data of all environment sensors in the residence (step S28: No), the consistency determination unit 131 adds "1" to "x" (step S29). After that, the consistency determination unit 131 proceeds to step S27.

On the other hand, when it is determined that there is a consistency between the motion sensor data of the subject and the changes in the data of all environment sensors in the residence (step S28: Yes), the change extraction unit 132 extracts a change in the data of the environment sensor which does not react to any person other than the subject (step S30). In other words, the change extraction unit 132 extracts a change in the data of the environment sensor that reacts to the subject only. For example, the change extraction unit 132 determines whether there is a consistency between the motion sensor data and the data obtained by an environment sensor when the environment sensor was caused to react to a person other than the subject using the temporarily stored data. The change extraction unit 132 extracts a change in the data of the environment sensor for which it is determined that no consistency is found even when the environment sensor was caused to react to any person other than the subject.

Subsequently, the segment specifying unit 13A specifies a behavior segment and a non-behavior segment using the extracted change in the environment sensor data (step S31).

The segment specifying unit 13A determines whether all items of the input one-day data have been read (step S32). When it is determined that all items of the input one-day data have not been read (step S32: No), the segment specifying unit 13A proceeds to step S21 so that the next data is read. On the other hand, when it is determined that all items of the input one-day data have been read (step S32: Yes), the segment specifying unit 13A ends the segment specifying process.

### [Specific Example of Consistency Determination Process]

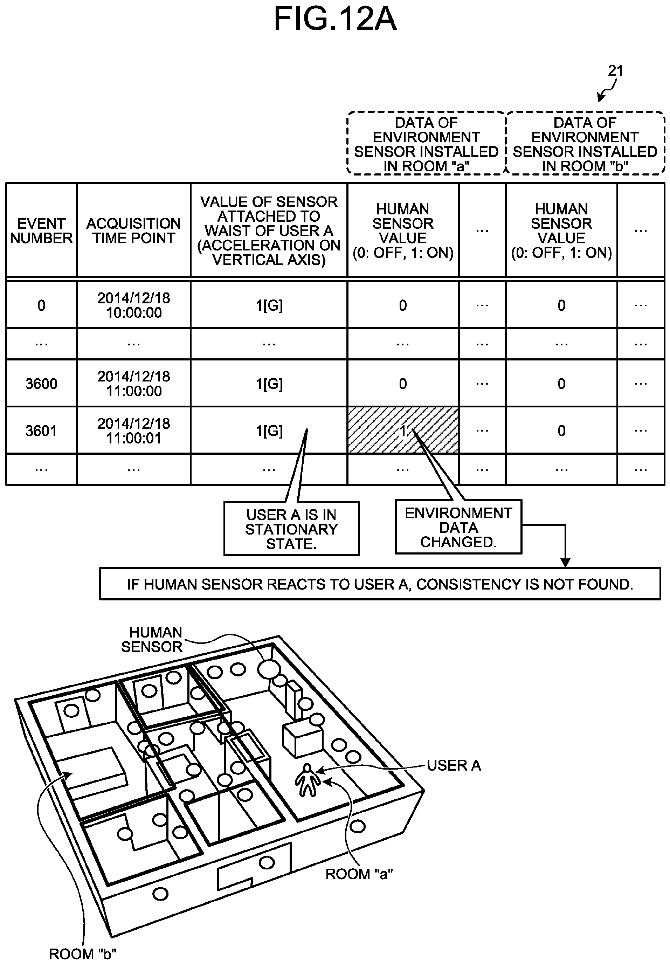

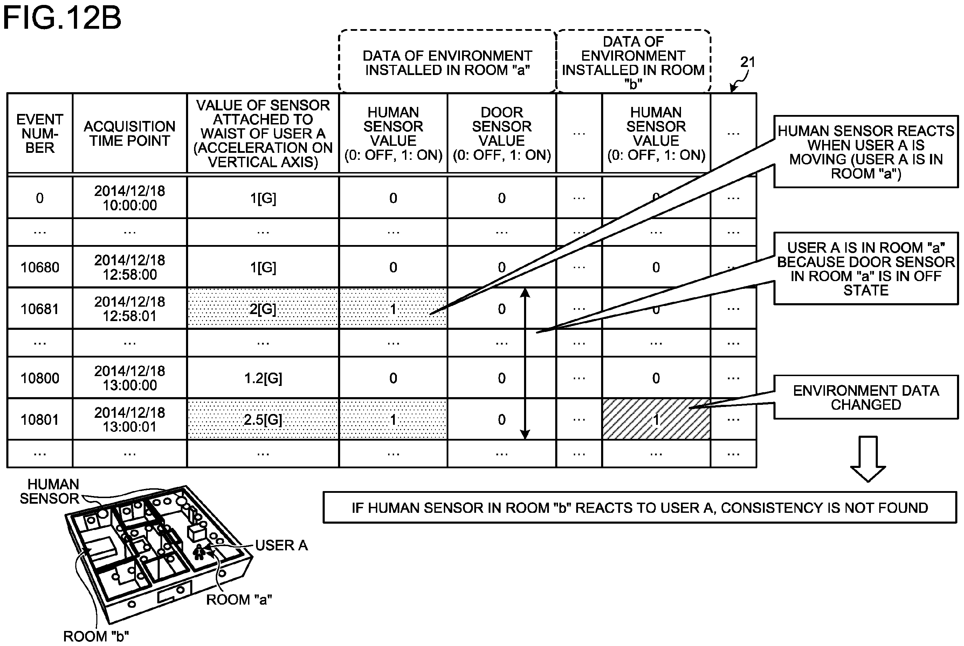

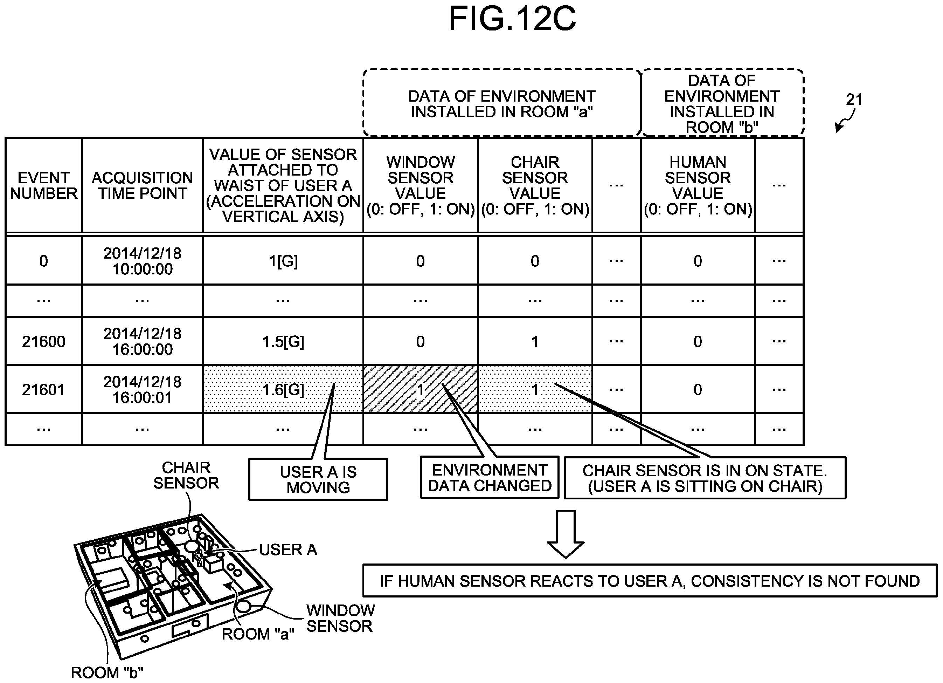

Next, a specific example of a consistency determination process according to the second embodiment will be described with reference to FIGS. 12A to 12C. FIG. 12A illustrates a case in which there is no consistency and there is a change in the environment sensor data when there is no reaction of the motion sensor and corresponds to FIG. 7. FIG. 12B illustrates a case in which there is no consistency and there is a change in the data of an environment sensor in a room different from the room where the subject is present and corresponds to FIG. 8. FIG. 12C illustrates a case in which there is no consistency and there is a change in the data of the environment sensor in a room where the subject is present even when the subject does not stand up after being seated and corresponds to FIG. 9. In FIGS. 12A to 12C, the subject will be described as a user A. Moreover, it is assumed that the motion sensor is attached to the waist of the user and the motion sensor value is the value of an acceleration on the vertical axis.

As illustrated in FIG. 12A, the value of a motion sensor attached to the waist of the user A and the value of a human sensor installed in the room "a" are stored in the data recording DB 21. The consistency determination unit 131 determines whether there is a consistency between the motion sensor data and the changes in the data of all environment sensors using the data stored in the data recording DB 21. In this example, when the event number is "3601", although the value of the human sensor installed in the room "a" changes from 0 (OFF) to 1 (ON), the value of the motion sensor of the user A is "1 [G]" (a stationary state). If the human sensor reacts to the user A, no consistency is found. That is, the consistency determination unit 131 determines that there is no consistency between the motion sensor of the user A and the human sensor in the room "a". Moreover, the consistency determination unit 131 determines that there is another person other than the user A and adds "1" to the number of persons.

As illustrated in FIG. 12B, the value of the motion sensor attached to the waist of the user A, the values of the human sensor and the door sensor installed in the room "a", and the value of the human sensor installed in the room "b" are stored in the data recording DB 21. The consistency determination unit 131 determines whether there is a consistency between the motion sensor data and the changes in the data of all environment sensors using the data stored in the data recording DB 21. In this example, when the event number is "10681", the value of the motion sensor of the user A is "2 [G]" and the user A is moving. Since the human sensor value changes from 0 (OFF) to 1 (ON) when the user A is moving, the room "a" associated with the human sensor is specified as a room where the user A is present. For event numbers of "10681" and later, since the door sensor value remains at 0 (OFF), the user A is in the room "a". When the event number is "10801", although the value of the human sensor in the room "b" different from the room "a" where the user A is present changes from 0 (OFF) to 1 (ON), no consistency is found if the human sensor in the room "b" reacts to the user A. That is, the consistency determination unit 131 determines that there is no consistency between the motion sensor of the user A and the human sensor in the room "b". Moreover, the consistency determination unit 131 determines that there is another person other than the user A and adds "1" to the number of persons.

As illustrated in FIG. 12C, the value of the motion sensor attached to the waist of the user A, the values of the window sensor and the chair sensor installed in the room "a", and the value of the human sensor installed in the room "b" are stored in the data recording DB 21. The consistency determination unit 131 determines whether there is a consistency between the motion sensor data and the changes in the data of all environment sensors using the data stored in the data recording DB 21. In this example, when the event number is "21601", the value of the motion sensor of the user A is "1.6 [G]" and the value of the chair sensor in the room "a" remains at 1 (ON). Therefore, the user A is in a "seated" state of being seated on a chair. In this case, although the value of the window sensor in the room "a" changes from 0 (OFF) to 1 (ON), no consistency is found if the window sensor in the room "a" reacts to the user A. That is, the consistency determination unit 131 determines that there is no consistency between the motion sensor of the user A and the window sensor in the room "a". Moreover, the consistency determination unit 131 determines that there is another person other than the user A and adds "1" to the number of persons.

### [Specific Example of Consistency Determination Process]

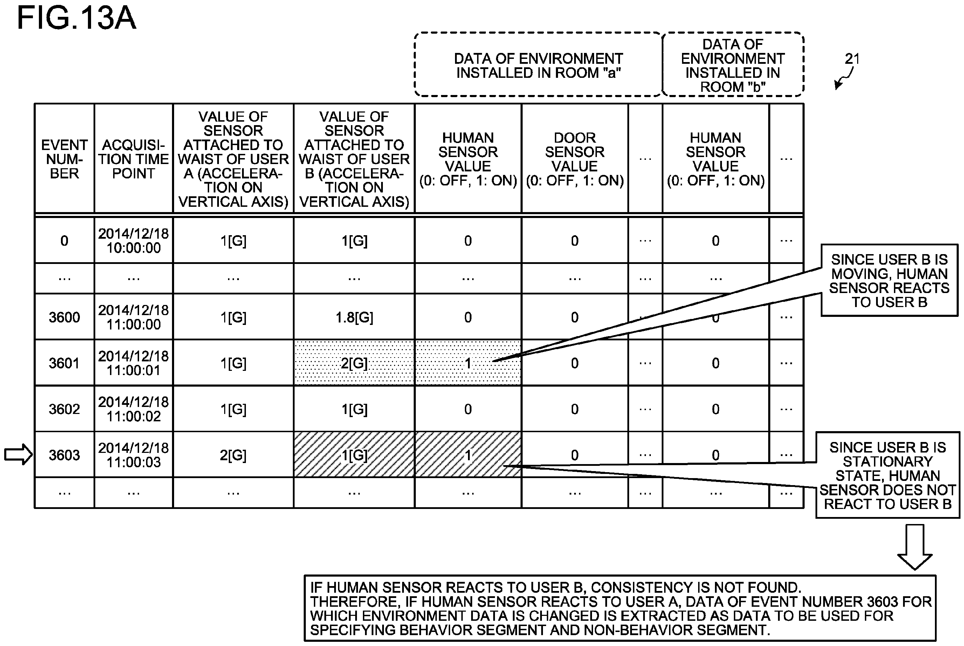

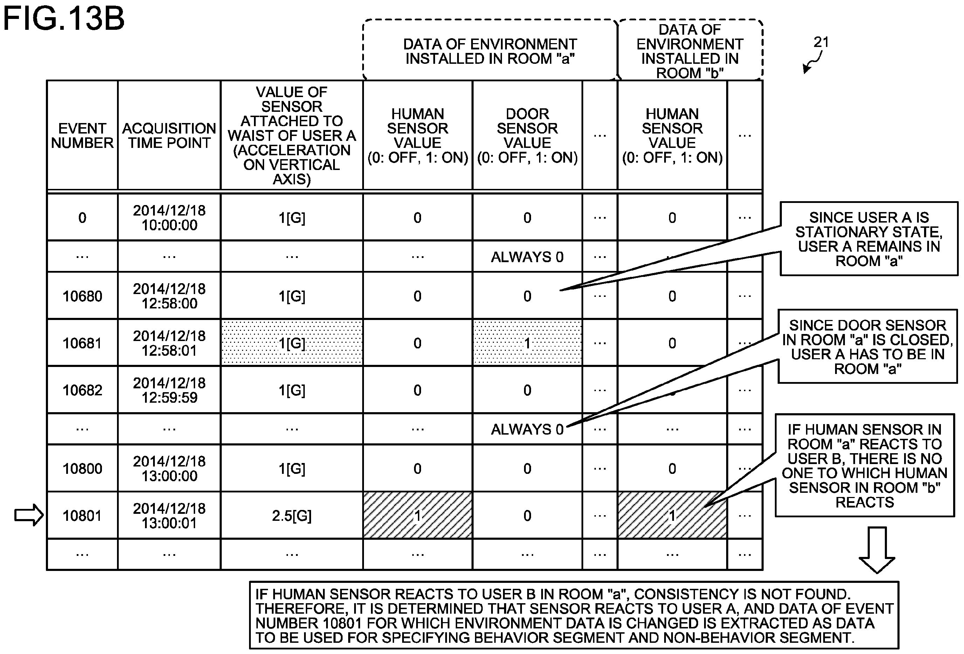

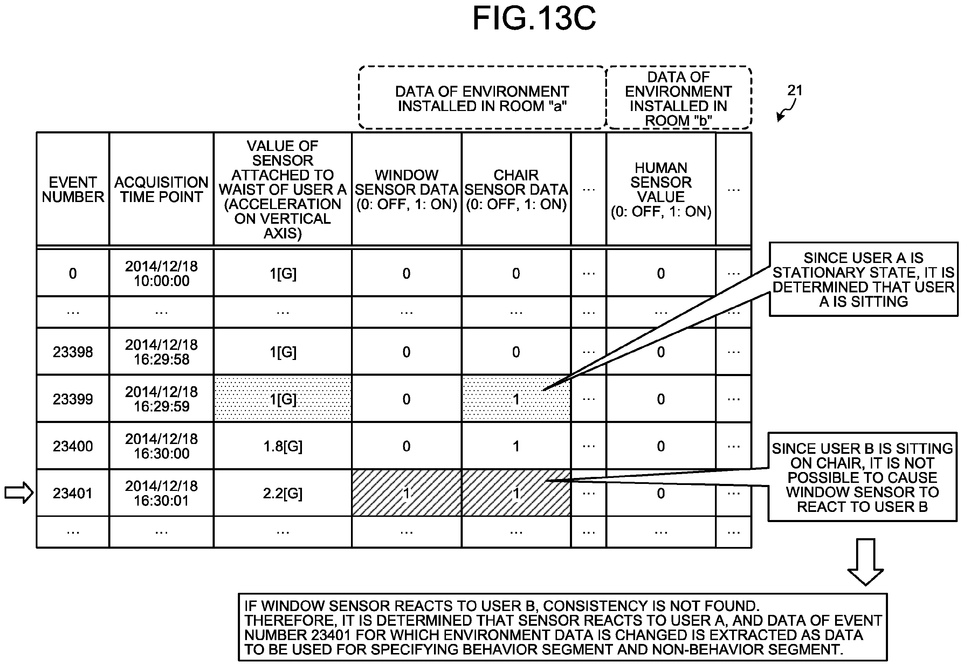

Next, a specific example of a change extraction process according to the second embodiment will be described with reference to FIGS. 13A to 13C. FIG. 13A illustrates a case in which the data of an environment sensor changes when there is no reaction of a motion sensor attached to the user B. FIG. 13B illustrates a case in which there is a change in the data of an environment sensor in a room different from the room where the user B is present. FIG. 13C illustrates a case in which there is a change in the data of an environment sensor in a room where the user A is present even when the user B does not stand up after being seated. In FIGS. 13A to 13C, the subject will be described as a user A. Moreover, it is assumed that the motion sensor is attached to the waist of the user and the motion sensor value is the value of an acceleration on the vertical axis.

As illustrated in FIG. 13A, the value of a motion sensor attached to the waist of the user B and the value of a human sensor installed in the room "a" are stored in the data recording DB 21. The change extraction unit 132 determines whether there is a consistency between the data of a motion sensor of a person other than the subject and changes in the data of all environment sensors for persons other than the subject using the data stored in the data recording DB 21. In this example, when the event number is "3603", although the value of the human sensor installed in the room "a" changes from 0 (OFF) to 1 (ON), the value of the motion sensor of the user B is "1 [G]" (a stationary state). If the human sensor reacts to the user B, no consistency is found. Therefore, the change extraction unit 132 determines that the human sensor in the room "a" reacts to the user A. Therefore, the change extraction unit 132 extracts the data of the event number 3603 indicating the change in the data of the human sensor in the room "a", for which it is determined that the human sensor data is not consistent with the user B as data to be used for specifying a behavior segment and a non-behavior segment.

As illustrated in FIG. 13B, the value of the motion sensor attached to the waist of the user A, the value of the human sensor installed in the room "a", and the value of the human sensor installed in the room "b" are stored in the data recording DB 21. The change extraction unit 132 determines whether there is a consistency between the data of a motion sensor of a person other than the subject and changes in the data of all environment sensors for persons other than the subject using the data stored in the data recording DB 21. In this example, it is determined that the user A is present in the room "a" based on the data recording DB 21. When the event number is "10801", the value of the human sensor in the room "b" different from the room "a" where the user A is present changes from 0 (OFF) to 1 (ON), and the value of the human sensor in the room "a" where the user A is present changes from 0 (OFF) to 1 (ON). If the human sensor in the room "a" reacts to the user B, no consistency is found. Therefore, it is determined that the human sensor in the room "a" reacts to the user A. Therefore, the change extraction unit 132 extracts the data of the event number 10801 indicating the change in the data of the human sensor in the room "a", for which it is determined that the human sensor data is not consistent with the user B as data to be used for specifying a behavior segment and a non-behavior segment.

As illustrated in FIG. 13C, the value of the motion sensor attached to the waist of the user A, the values of the window sensor and the chair sensor installed in the room "a", and the value of the human sensor installed in the room "b" are stored in the data recording DB 21. The change extraction unit 132 determines whether there is a consistency between the data of a motion sensor of a person other than the subject and changes in the data of all environment sensors for persons other than the subject using the data stored in the data recording DB 21. In this example, when the event number is "23399", since the value of the motion sensor of the user A is "1 [G]" (a stationary state) and the value of the chair sensor in the room "a" changes from 0 (OFF) to 1 (ON), it is determined that the user B is seated on the chair in the room "a". When the event number is "23401", although the value of the window sensor in the room "a" changes from 0 (OFF) to 1 (ON), since the user B is in a "seated" state of being seated on a chair, it is not possible to cause the window sensor in the room "a" to react to the user B. That is, no consistency is found if the window sensor in the room "a" reacts to the user B. Therefore, it is determined that the window sensor in the room "a" reacts to the user A. Therefore, the change extraction unit 132 extracts the data of the event number 23401 indicating the change in the data of the window sensor in the room "a", for which it is determined that the window sensor data is not consistent with the user B as data to be used for specifying a behavior segment and a non-behavior segment.

### [Advantages of the second embodiment]

In this way, in the second embodiment, the state detection device 1 estimates the number of persons present in a space where environment sensors are present using the data obtained from a motion sensor and the data obtained from environment sensors, stored in the data recording DB 21. The state extraction device 1 extracts a change in the data which is not changed by a person other than the subject from the data obtained from the environment sensors, stored in the data recording DB 21. The state extraction device 1 specifies a behavior space and a non-behavior space using the extracted change in the data. According to this configuration, the state extraction device 1 can extract a change in the data changed by the subject by extracting the change in the data that is not changed by other persons even when there is another person other than the subject and to specify a behavior space and a non-behavior space of the subject. As a result, the state extraction device 1 can determine a threshold value of a specific state depending on the current situation of the subject even when there is another person other than the subject.

### Third Embodiment

A case in which the state detection devices 1 according to the first and second embodiments determine the feature amount and the threshold value to be used for detecting walking of a subject using the respective values of the respective feature amounts of a motion sensor in each of the behavior segment and the non-behavior segment has been described. However, when a change in the threshold value matches a predetermined pattern, the state detection device 1 may estimate a threshold value in a predetermined subsequent segment according to the predetermined matching pattern. The predetermined pattern may be a monotonously increasing or decreasing pattern, for example.

Therefore, a case in which when a change in the threshold value matches a predetermined pattern, the state detection device 1 according to a third embodiment estimates a threshold value in a predetermined subsequent segment according to the predetermined matching pattern.

### [Configuration of State Detection Device according to the third embodiment]

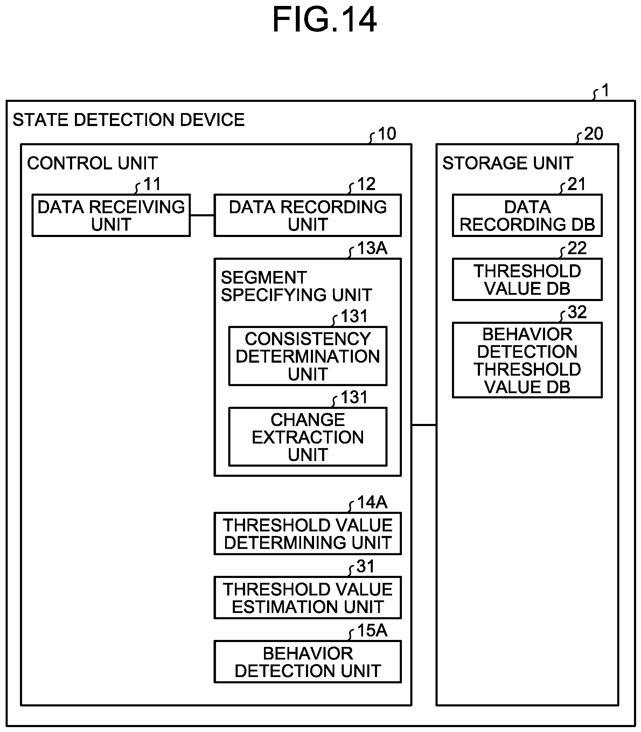

FIG. 14 is a functional block diagram illustrating a configuration of a state detection device according to the third embodiment. The same constituent elements as those of the state detection device 1 illustrated in FIG. 6 are denoted by the same reference numerals, and the redundant description of the constituent elements and operations will not be provided. A difference between the second embodiment and the third embodiment is that the threshold value determining unit 14 is changed to a threshold value determining unit 14A and the behavior detection unit 15 is changed to a behavior detection unit 15A. Another difference between the second embodiment and the third embodiment is that a threshold value estimation unit 31 is added to the control unit 10. Another difference between the second embodiment 2 and the third embodiment is that a behavior detection threshold value DB 32 is added to the storage unit 20.

The threshold value determining unit 14A determines a feature amount and a threshold value to be used for detecting walking of a subject using respective values of respective feature amounts of a motion sensor, stored in the data recording DB 21 in each of a behavior segment and a non-behavior segment in a predetermined segment. Moreover, the threshold value determining unit 14A adds the determined threshold value and the determined feature amount to the threshold value DB 22 in correlation with the predetermined segment when the threshold value and the feature amount were determined. The predetermined segment is one day, for example, but is not limited to this and may be a half day or a 3/4 day. That is, the predetermined segment may be a segment in which a threshold value can be determined. Hereinafter, a case in which the predetermined segment is one day will be described.

When a change in the threshold value matches a predetermined pattern, the threshold value estimation unit 31 estimates a threshold value in the next one day according to the predetermined matching pattern. In other words, the threshold value estimation unit 31 assumes that a feature amount of which the threshold value monotonously increases (decreases) day by day and the amount of change in each day is large among the feature amounts to be used for behavior detection has the same tendency on the next day and estimates a threshold value to be used for the next day for the feature amount. For example, the threshold value estimation unit 31 determines whether a change in a threshold value obtained from the evaluation values of every day, stored in the threshold value DB 22 matches a predetermined pattern. When it is determined that the change in the threshold value matches the predetermined pattern, the threshold value estimation unit 31 estimates the threshold value of the next one day from the amount of change in the threshold value. Moreover, the threshold value estimation unit 31 overwrites the estimated threshold value in the behavior detection threshold value DB 32 together with the feature amount determined in advance. In this way, the threshold value estimation unit 31 can determine a threshold value with high accuracy depending on the current situation of the subject by estimating the threshold value of the next one day according to the changing pattern of the threshold value.

The behavior detection unit 15A detects walking of the subject using the threshold value and the feature amount stored in the behavior detection threshold value DB 32 from the information obtained from the motion sensor. In this way, the behavior detection unit 15A can detect walking as a behavior type with high accuracy depending on the current situation of the subject.

### [Example of Threshold Value Estimation Process]

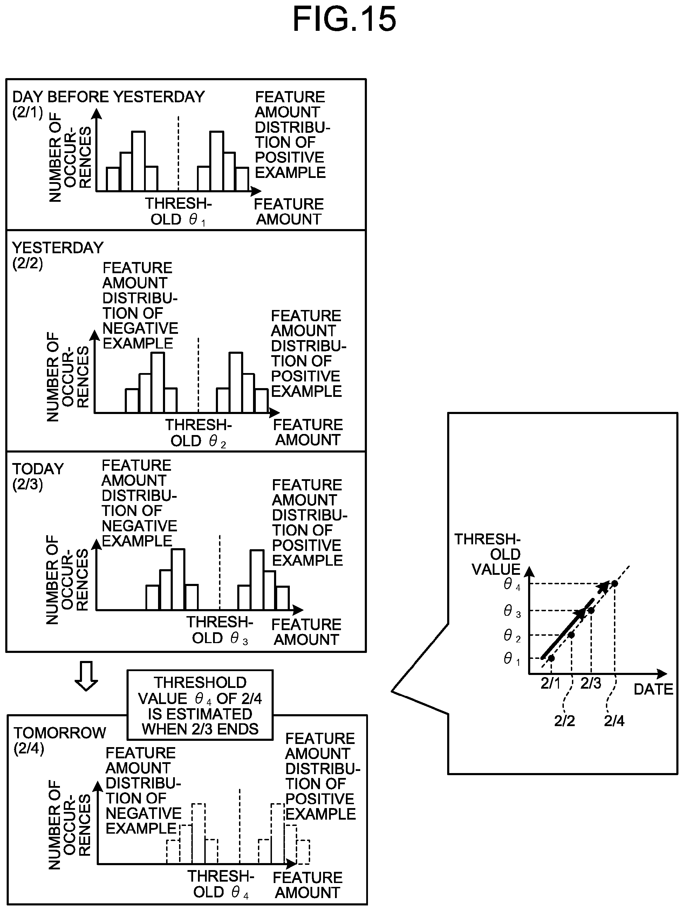

FIG. 15 is a diagram illustrating an example of a threshold value estimation process according to the third embodiment. As illustrated in FIG. 15, it is assumed that, if today is February 3 (2/3), the threshold value determining unit 14A determines that a threshold value of February 1 (2/1) which is the day before yesterday is θ₁ when the threshold value estimation process on January 31 (1/31) ends. It is also assumed that the threshold value determining unit 14A determines that a threshold value of February 2 (2/2) which is the yesterday is θ₂ when the threshold value estimation process on the date of 2/1 ends. It is also assumed that the threshold value determining unit 14A determines that a threshold value of today 2/3 is θ₃ when the threshold value estimation process on the date 2/2 ends.

When the threshold value of February 4 (2/4) is determined when the threshold value estimation process on the date 2/3 ends and when the change in the threshold values accumulated every previous day matches a predetermined pattern, the threshold value estimation unit 31 estimates the threshold value of the next one day according to the predetermined matching pattern. In this example, the threshold value estimation unit 31 determines that the change in the threshold value matches a monotonously increasing pattern. The threshold value estimation unit 31 estimates the threshold value θ₄ of the date 2/4 from the amount of change in the threshold value. For example, the threshold value estimation unit 31 may calculate a linear model from the amount of change in the threshold value and estimate the threshold value of the date 2/4.

Although it has been described that the threshold value estimation unit 31 estimates the threshold value of the next one day from the amount of change in the threshold value when the change in the threshold value matches a monotonously increasing or decreasing pattern, for example, the present invention is not limited to this. The threshold value estimation unit 31 may estimate the threshold value of the next one day from the amount of change in the threshold value when the change in the threshold value matches a monotonously increasing or decreasing pattern and the amount of change in each day is larger than a specific amount.

### [Flowchart of Threshold Value Estimation Process]

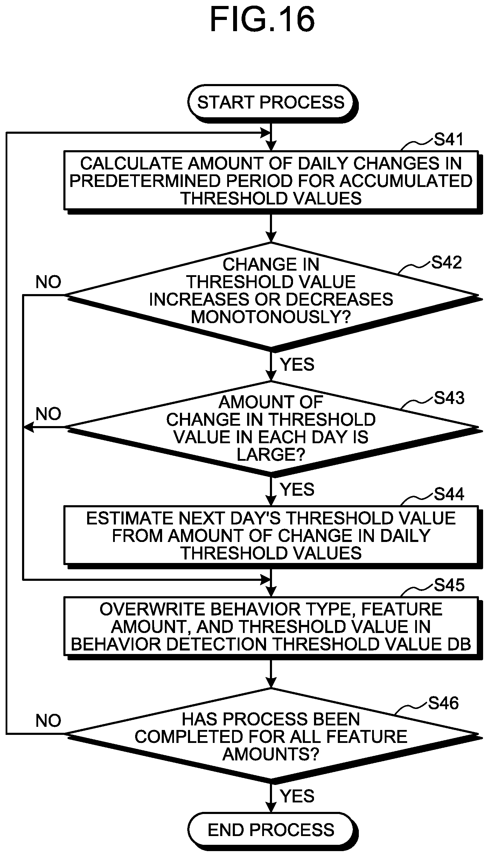

Next, an example of a processing procedure of a threshold value estimation process according to the third embodiment will be described with reference to FIG. 16. FIG. 16 is a flowchart illustrating a processing procedure of a threshold value estimation process according to the third embodiment. A processing procedure of an entire state detection process including the segment specifying process is the same as that described in FIGS. 5 and 11, and the description thereof will not be provided. An example of S16 in the processing procedure of the state detection process described in FIG. 5 is a processing procedure of the threshold value estimation process illustrated in this diagram.

In this example, a threshold value DB 22 determined by the threshold value determining unit 14A every day is accumulated in correlation with a feature amount and a behavior type. The behavior type is "walking" as an example and is a type for distinguishing subject's behaviors that are to be detected.

The threshold value estimation unit 31 performs a threshold value estimation process for each of the feature amounts accumulated in the threshold value DB 22. First, the threshold value estimation unit 31 calculates the amount of daily change in a predetermined period with respect to the threshold value accumulated in the threshold value DB 22 (step S41). The threshold value estimation unit 31 determines whether the change in the threshold value is monotonously increasing or decreasing based on the calculated amount of daily change in a predetermined period (step S42). That is, the threshold value estimation unit 31 determines whether the change in the threshold value matches a predetermined pattern.

When it is determined that the change in the threshold value is not monotonously increasing or decreasing (step S42: No), the threshold value estimation unit 31 proceeds to step S45 in order to determine the threshold value determined by the threshold value determining unit 14A as a threshold value for behavior detection. On the other hand, when it is determined that the change in the threshold value is monotonously increasing or decreasing (step S42: Yes), the threshold value estimation unit 31 determines whether the amount of change in the threshold value for each day is large (step S43) .

When it is determined that the amount of change in the threshold value for each day is not large (step S43: No), the threshold value estimation unit 31 proceeds to step S45 in order to determine the threshold value determined by the threshold value determining unit 14A as a threshold value for behavior detection. On the other hand, when it is determined that the amount of change in the threshold value for each day is large (step S43: Yes), the threshold value estimation unit 31 estimates the threshold value of the next day from the amount of daily change in the threshold value stored in the threshold value DB 22 (step S44). For example, the threshold value estimation unit 31 calculates a linear model from the amount of daily change in the threshold value stored in the threshold value DB 22 and estimates the threshold value of the next day.

The threshold value estimation unit 31 overwrites the behavior type, the feature amount, and the threshold value in the behavior detection threshold value DB 32 (step S45). The overwritten threshold value is an estimated threshold value when the threshold value is estimated by the threshold value estimation unit 31 and is a threshold value determined by the threshold value determining unit 14A when the threshold value is not estimated by the threshold value estimation unit 31.

The threshold value estimation unit 31 determines whether the threshold value estimation process has been completed for all feature amounts (step S46). When it is determined that the threshold value estimation process has not been completed for all feature amounts (step S46: No), the threshold value estimation unit 31 proceeds to step S41 in order to perform the threshold value estimation process for the subsequent feature amount.

When it is determined that the threshold value estimation process has been completed for all feature amounts (step S46: Yes), the threshold value estimation unit 31 ends the threshold value estimation process.

### [Advantages of the third embodiment]

In this way, in the third embodiment, the state detection device 1 determines the threshold value for respective predetermined segments. When a change in the threshold value matches a predetermined pattern, the state detection device 1 estimates a threshold value in a subsequent predetermined segment. According to this configuration, the state detection device 1 can determine the threshold value to be used for detecting the specific state with high accuracy by estimating the threshold value in a subsequent predetermined segment using the threshold value determined in respective predetermined segments. As a result, the state detection device 1 can determine a specific state of the subject (for example, a behavior of the subject) with high accuracy depending on the current situation of the subject.

### [Others]

In the first to third embodiments, it has been described that the state detection device 1 has the behavior detection unit 15, and the behavior detection unit 15 detects the behavior of the subject from the information obtained from the motion sensor using the threshold value and the feature amount stored in the threshold value DB 22. However, the motion sensor may have a functional unit corresponding to the behavior detection unit 15 and a threshold value storage unit corresponding to the threshold value DB 22. The motion sensor receives the threshold value and the feature amount determined by the threshold value determining unit 14 of the state detection device 1 and records the received threshold value and the received feature amount in the threshold value storage unit. The functional unit corresponding to the behavior detection unit 15 may detect the behavior of the subject having a motion sensor attached thereto from the information obtained from the motion sensor using the threshold value and the feature amount stored in the threshold value storage unit. In this way, the state detection device 1 can detect the behavior of the subject at a high speed since communication with the motion sensor does not occur.

In the first to third embodiments, walking has been described as an example of the behavior type for which the threshold value changes depending on the situation of the subject. That is, the state detection device 1 determines a feature amount and a threshold value to be used for detecting walking of the subject and detects walking of the subject using the determined feature amount and the determined threshold value. However, the behavior type for which the threshold value changes depending on the situation of the subject may be "sitting" or "standing up". In such a case, for example, the segment specifying unit 13 may specify the behavior segment and the non-behavior segment of the subject using the values of a sensor (a chair sensor) installed in a chair as the environment sensor. Moreover, the threshold value determining unit 14 may determine the feature amount and the threshold value to be used for detecting "sitting" or "standing up" of the subject using the respective values of the respective feature amounts of the motion sensor in each of the behavior segment and the non-behavior segment. In this way, in a situation in which the subject sits or stands up slowly such as a case in which the subject is wounded or was discharged from a hospital, the state detection device 1 can detect "sitting" or "standing up" of the subject using the motion sensor of the subject depending on the situation of the subject.

The behavior type for which the threshold value changes depending on the situation of the subject may be ascending or descending stairs. In such a case, for example, the segment specifying unit 13 may specify that the behavior of the subject is ascending or descending of stairs using human sensors installed in an entrance as environment sensors and may specify the behavior segment and the non-behavior segment of the subject. Moreover, the threshold value determining unit 14 may determine the feature amount and the threshold value to be used for detecting the subject ascending or descending stairs using the respective values of the respective feature amounts of the motion sensor in each of the behavior segment and the non-behavior segment. In this way, in a case in which a person ascends or descends stairs slowly so as not to disturb other people, the state detection device 1 can detect the subject ascending or descending stairs using the motion sensor of the subject only depending on the situation of the subject.

The behavior type for which the threshold value changes depending on the situation of the subject may be opening or closing of a door. In such a case, for example, the segment specifying unit 13 may specify that the behavior of the subject is opening or closing of a door using a door sensor installed in a door as an environment sensor and may specify the behavior segment and the non-behavior segment of the subject. Moreover, the threshold value determining unit 14 may determine the feature amount and the threshold value to be used for detecting the subject opening or closing of a door using respective values of the respective feature amounts of the motion sensor in each of the behavior segment and the non-behavior segment. In this way, in a situation in which a person holds something in his or her hands and slow pushes a door slowly using a portion of the body, the state detection device 1 can detect the subject ascending or descending stairs using the motion sensor of the subject only depending on the situation of the subject.

In the first to third embodiments, it has been described that the state detection device 1 detects the feature amount and the threshold value to be used for detecting the behavior of the subject. However, the state detection device 1 may determine the feature amount and the threshold value to be used for detecting a biological state of the subject. Examples of the biological state include an abnormal disorder in electrocardiogram and an abnormally high heart rate. When the biological state is an abnormal disorder in electrocardiogram, for example, the segment specifying unit 13 specifies a segment (corresponding to a behavior segment) in which an abnormal disorder in electrocardiogram in comparison to the subject's behavior is present and a segment (corresponding to a non-behavior segment) in which a disorder in electrocardiogram is natural in comparison to the subject's behavior. Moreover, the threshold value determining unit 14 may determine the feature amount and the threshold value to be used for detecting an abnormal disorder in electrocardiogram of the subject using electrocardiogram obtained from an electrocardiogram detection sensor in each of the segment corresponding to the behavior segment and the segment corresponding to the non-behavior segment. In such a case, the feature amount may be a dispersion of the heights and the time intervals of electrocardiogram, a maximum-to-minimum ratio, and the like. In this way, the state detection device 1 can detect an abnormal disorder in electrocardiogram of the subject using the electrocardiogram of the subject only depending on the situation of the subject.

In the third embodiment, it has been described that the state detection device 1 determines the feature amount and the threshold value to be used for detecting the subject's behavior and detects the behavior of the subject using the determined threshold value and the determined feature amount. However, the state detection device 1 is not limited to this and may quantize the feature of the state in a period in which the detected subject's state continues. Examples of an index indicating the feature of a behavior when the state is walking include a cadence, a walking cycle, a variation in walking cycle, a stride, a variation in stride, a walking speed, and a foot-to-foot distance. The cadence means the number of steps per minutes. The walking cycle means a period after the heel of one foot contacts the ground before the heel of the other foot contacts the ground. A variation in walking cycle means the ratio of a standard deviation of the walking cycles and the average of the walking cycles ((standard deviation of walking cycles)/(average of walking cycles)). The stride means the distance from the tiptoe of one foot to the tiptoe of the other foot or the distance from the heel of one foot to the heel of the other foot. A variation in stride means the ratio of a standard deviation of stride to the average. A walking speed means a division of a walking distance from the start of walking to the end of walking by a walking period. The foot-to-foot distance means the distance between the left and right heels when the subject was seen from the top. The foot-to-foot distance is 0 when the subject walking along a straight line. In this way, the state detection device 1 can detect the walking state of the subject.

The constituent elements of each device illustrated do not always need to be physically configured as illustrated in the drawings. That is, specific forms of separation and integration of each device are not limited to those depicted in the drawings, and all or some of them may be functionally or physically separated or integrated in arbitrary units depending on various types of loads or usage. For example, the segment specifying unit 13 and the threshold value determining unit 14 may be integrated as a single unit. Moreover, the data recording DB 21 and the threshold value DB 22 may be connected via a network as external devices of the state detection device 1.

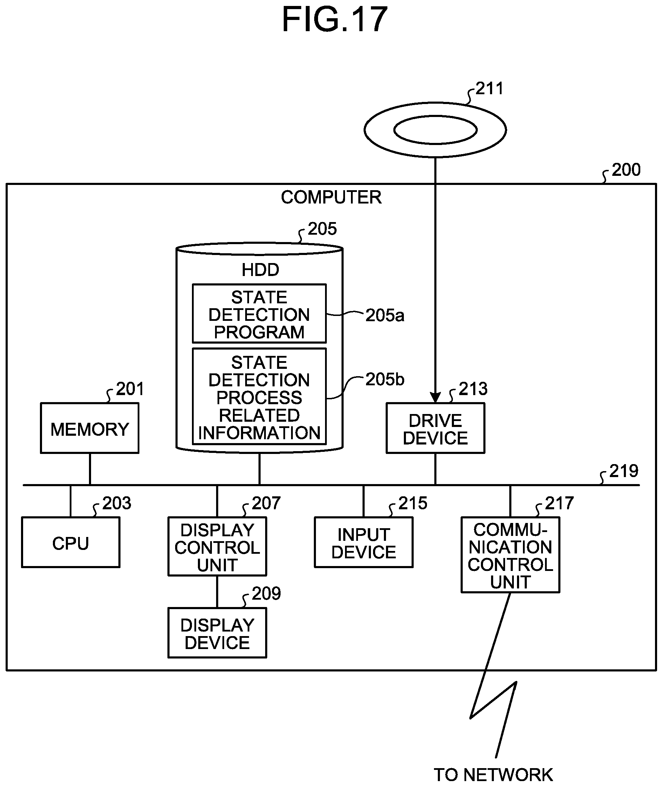

Various processes described in the above-described embodiments may be implemented when a computer such as a personal computer or a workstation executes a program prepared in advance. Hereinafter, an example of a computer that executes a state detection program that implements the same functions as the state detection device 1 illustrated in FIG. 1 will be described. FIG. 17 is a diagram illustrating an example of a computer that executes a state detection program.

As illustrated in FIG. 17, a computer 200 includes a CPU 203 that executes various arithmetic processes, an input device 215 that receives data input from users, and a display control unit 207 that controls a display device 209. Moreover, the computer 200 includes a drive device 213 that reads a program and the like from a storage medium and a communication control unit 217 that exchanges data with another computer via a network. Moreover, the computer 200 includes a memory 201 that temporarily stores various items of information and a HDD 205. The memory 201, the CPU 203, the HDD 205, the display control unit 207, the drive device 213, the input device 215, and the communication control unit 217 are connected by a bus 219.

The drive device 213 is a device for a removable disk 211, for example. The HDD 205 stores a state detection program 205a and state detection process related information 205b.

The CPU 203 reads the state detection program 205a, loads the program into the memory 201, and executes the program as a process. The process corresponds to each functional unit of the state detection device 1. The state detection process related information 205b corresponds to the data recording DB 21 and the threshold value DB 22. For example, the removable disk 211 stores items of information such as the state detection program 205a.

The state detection program 205a is not necessarily stored initially in the HDD 205. For example, the program may be stored in a "portable physical medium" such as a flexible disk (FD), a CD-ROM, a DVD disc, an opto-magnetic disk, or an IC card inserted into the computer 200. The computer 200 may reads the state detection program 205a from these media and execute the program.

### [Explanation of Reference]

1 STATE DETECTION DEVICE
10 CONTROL UNIT
11 DATA RECEIVING UNIT
12 DATA RECORDING UNIT
13, 13A SEGMENT SPECIFYING UNIT
14, 14A THRESHOLD VALUE DETERMINING UNIT
15, 15A BEHAVIOR DETECTION UNIT
131 CONSISTENCY DETERMINATION UNIT
132 CHANGE EXTRACTION UNIT
20 STORAGE UNIT
21 DATA RECORDING DB
22 THRESHOLD VALUE DB
31 THRESHOLD VALUE ESTIMATION UNIT
32 BEHAVIOR DETECTION THRESHOLD VALUE DB

## Claims

1. A state detection method for causing a computer to execute processes comprising:
storing first information obtained from a first sensor and second information obtained from a second sensor in a predetermined segment in a storage unit;
specifying a behavior segment indicating a segment in which a subject performs a specific behavior and a non-behavior segment indicating a segment in which the subject does not perform the specific behavior using the second information stored in the storage unit;
determining a feature amount to be used for detecting a specific state of the subject using respective values of a plurality of feature amounts included in the first information stored in the storage unit in each of the behavior segment and the non-behavior segment and determining a threshold value for the determined feature amount, for distinguishing a behavior and a non-behavior; and
detecting the specific state of the subject from the information obtained from the first sensor using the determined feature amount and the determined threshold value.

2. The state detection method according to claim 1, wherein the specifying process executing processes comprising:
estimating the number of persons present in a space where the second sensor is present using the first information and the second information stored in the storage unit; and
extracting a change in data which is not changed by a person other than the subject from the second information stored in the storage unit and specifying the behavior segment and the non-behavior segment using the extracted change in the data.

3. The state detection method according to claim 2, wherein the estimating process executing a process comprising:
estimating a smallest number of persons which satisfies consistency with a change in the data extracted from the first information and the second information as the number of persons present in the space where the second sensor is present.

4. The state detection method according to any one of claims 1 to 3, wherein the determining process executing a process comprising:
determining the threshold value in respective predetermined segments and estimating the threshold value in a subsequent predetermined segment according to a predetermined pattern when a change in the threshold value matches the predetermined pattern.

5. The state detection method according to claim 1, wherein the specific state is a behavior of the subject or a biological state of the subject.

6. The state detection method according to claim 1, wherein the second sensor includes a human sensor or a temperature and humidity sensor that measures a surrounding environment, or a door sensor, a window sensor, a chair sensor, or a bed sensor that measures the state of an attached object itself or any combination thereof.

7. The state detection method according to claim 1, wherein the first sensor includes an acceleration sensor or a gyro sensor that measures a motion of the subject, or an electrocardiogram sensor or a pulse sensor that measures a biological state of the subject or any combination thereof.

8. The state detection method according to claim 1, wherein the space where the second sensor is present is a closed environment space.

9. The state detection method according to claim 1, wherein an index indicating a feature of a state is calculated in a period in which the detected specific state of the subject continues.

10. A state detection device comprising:
a recording unit configured to record first information obtained from a first sensor and second information obtained from a second sensor in a predetermined segment in a storage unit;
a specifying unit configured to specify a behavior segment indicating a segment in which a subject performs a specific behavior and a non-behavior segment indicating a segment in which the subject does not perform the specific behavior using the second information recorded in the recording unit;
a determining unit configured to determine a feature amount to be used for detecting a specific state of the subject using respective values of a plurality of feature amounts included in the first information stored in the storage unit in each of the behavior segment and the non-behavior segment and determine a threshold value for the determined feature amount, for distinguishing a behavior and a non-behavior; and
a detecting unit configured to detect the specific state of the subject from the information obtained from the first sensor using the feature amount and the threshold value determined by the determining unit.

11. A state detection program that causes a computer to execute processes comprising:
storing first information obtained from a first sensor and second information obtained from a second sensor in a predetermined segment in a storage unit;
specifying a behavior segment indicating a segment in which a subject performs a specific behavior and a non-behavior segment indicating a segment in which the subject does not perform the specific behavior using the second information stored in the storage unit;
determining a feature amount to be used for detecting a specific state of the subject using respective values of a plurality of feature amounts included in the first information stored in the storage unit in each of the behavior segment and the non-behavior segment and determining a threshold value for the determined feature amount, for distinguishing a behavior and a non-behavior; and
detecting the specific state of the subject from the information obtained from the first sensor using the determined feature amount and the determined threshold value.
